(19) Europäisches Patentamt
European Patent Office
Office européen des brevets

(11) EP 1 533 373 A1

(12) # EUROPEAN PATENT APPLICATION
published in accordance with Art. 158(3) EPC

(43) Date of publication:
**25.05.2005 Bulletin 2005/21**

(21) Application number: **03730542.2**

(22) Date of filing: **21.05.2003**

(51) Int Cl.[7]: **C12N 15/09**, C12Q 1/68

(86) International application number:
**PCT/JP2003/006330**

(87) International publication number:
**WO 2003/097830 (27.11.2003 Gazette 2003/48)**

(84) Designated Contracting States:
**AT BE BG CH CY CZ DE DK EE ES FI FR GB GR HU IE IT LI LU MC NL PT RO SE SI SK TR**

(30) Priority: **22.05.2002 JP 2002148339**

(71) Applicants:
- **Japan Represented by President of the University of Tokyo**
**Bunkyo-ku, Tokyo 113-8654 (JP)**
- **SRL, INC.**
**Tachikawa-shi, Tokyo 190 (JP)**

(72) Inventors:
- **OHNO, Ryozo c/o Aichi Cancer Center**
**Nagoya-shi, Aichi 464-8681 (JP)**
- **TSURUO, Takashi c/o The University of Tokyo**
**Bunkyo-ku, Tokyo 113-0032 (JP)**
- **NAKAMURA, Yusuke;**
**c/o The University of Tokyo**
**Minato-ku, Tokyo 108 (JP)**

(74) Representative: **Tollervey, Rebecca Marie**
**Mewburn Ellis LLP,**
**York House,**
**23 Kingsway**
**London WC2B 6HP (GB)**

(54) **METHOD OF JUDGING SENSIBILITY TO IMATINIB**

(57) A method of judging whether a patient is sensitive to imatinib or not, in case where the patient is suffering from a disease such as CML to be treated by administration of imatinib, that is, a method for judging whether the administration of imatinib is effective for the therapy of the disease or not, is disclosed. Amounts of a plurality of genes selected from the group consisting of the specific 77 genes in sample cells separated from body are measured; and the measured amounts are compared with those of responders and non-responders to imatinib or a derivative thereof or a pharmaceutically acceptable salt thereof:

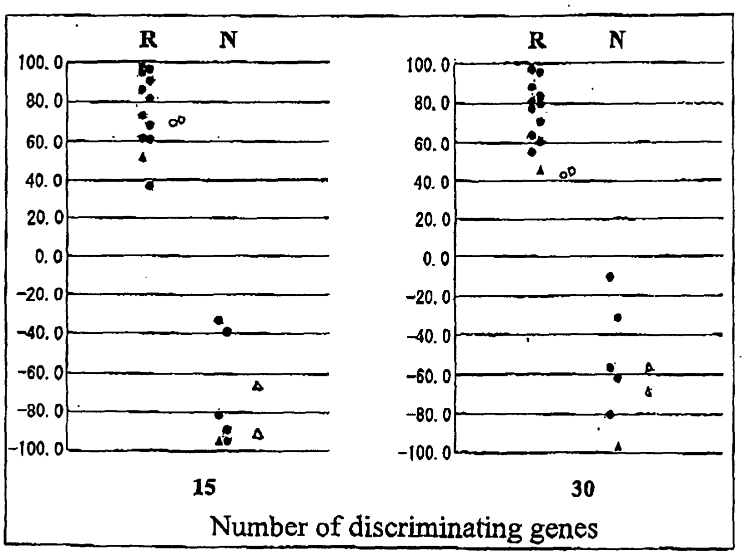


Fig. 4

EP 1 533 373 A1

SEQUENCE LISTING

<110> JAPAN REPRESENTED BY PRESIDENT OF THE UNIVERSITY OF TOKYO

<120> Method for Evaluating Sensitivity to Imatinib

<130> 03PF262-PCT

<160> 154

<210> 1

<211> 23

<212> DNA

<213> Artificial Sequence

<220>

<223> Oligonucleotide forward primer used for amplifying human HN1 cDNA

<400> 1

acccatagta ggcctaaaag cag 23

<210> 2

<211> 23

<212> DNA

<213> Artificial Sequence

<220>

<223> Oligonucleotide reverse primer used for amplifying human HN1 cDNA

<400> 2

tgggttgaca gtgagggtaa taa 23

<210> 3

<211> 23

<212> DNA

<213> Artificial Sequence

<220>

<223> Oligonucleotide forward primer used for amplifying human AKR1C3 cDNA

<400> 3

gttccgccat atagattctg ctc 23

<210> 4

<211> 24

<212> DNA

<213> Artificial Sequence

<220>

<223> Oligonucleotide reverse primer used for amplifying human AKR1C3 cDNA

<400> 4

ttattgtctt tctggcctat ggac 24

<210> 5

<211> 23

<212> DNA

<213> Artificial Sequence

<220>

<223> Oligonucleotide forward primer used for amplifying human QARS cDNA

<400> 5

gttccgccat atagattctg ctc 23

<210> 6

<211> 24

<212> DNA

<213> Artificial Sequence

<220>

<223> Oligonucleotide reverse primer used for amplifying human QARS cDN A

<400> 6

ttattgtctt tctggcctat ggac 24

<210> 7

<211> 23

<212> DNA

<213> Artificial Sequence

<220>

<223> Oligonucleotide forward primer used for amplifying human KIAA1105 cDNA

<400> 7

gagtaaatag accaaagtgg ggg 23

<210> 8

<211> 23

<212> DNA

<213> Artificial Sequence

<220>

<223> Oligonucleotide reverse primer used for amplifying human KIAA1105 cDNA

<400> 8

taacatcctt acacctttgc tcc 23

<210> 9

<211> 23

<212> DNA

<213> Artificial Sequence

<220>

<223> Oligonucleotide forward primer used for amplifying human KIAA0668 cDNA

<400> 9

gacaaaagtg tgtatttgtc ccg 23

<210> 10

<211> 23

<212> DNA

<213> Artificial Sequence

<220>

<223> Oligonucleotide reverse primer used for amplifying human KIAA0668 cDNA

<400> 10

caatagtgca gcatttgtga cag 23

<210> 11

<211> 23

<212> DNA

<213> Artificial Sequence

<220>

<223> Oligonucleotide forward primer used for amplifying human BLCAP cDNA

<400> 11

ttcagcaggc tagacagaaa cat 23

<210> 12

<211> 23

<212> DNA

<213> Artificial Sequence

<220>

<223> Oligonucleotide reverse primer used for amplifying human BLCAP cDNA

<400> 12

gaatagcagt ccagacgttt cac 23

<210> 13

<211> 23

<212> DNA

<213> Artificial Sequence

<220>

<223> Oligonucleotide forward primer used for amplifying human ADFP cDNA

<400> 13

tacctctcat gggtagagtg gaa 23

<210> 14

<211> 23

<212> DNA

<213> Artificial Sequence

<220>

<223> Oligonucleotide reverse primer used for amplifying human ADFP cDN

A

<400> 14

ggaggctgtc agacacttct tta 23

<210> 15

<211> 22

<212> DNA

<213> Artificial Sequence

<220>

<223> Oligonucleotide forward primer used for amplifying human FLJ10422 cDNA

<400> 15

gagattatgt tgcaaatggt gg 22

<210> 16

<211> 22

<212> DNA

<213> Artificial Sequence

<220>

<223> Oligonucleotide reverse primer used for amplifying human FLJ10422 cDNA

<400> 16

gctcctcgaa tgacaatcag tt 22

<210> 17

<211> 21

<212> DNA

<213> Artificial Sequence

<220>

<223> Oligonucleotide forward primer used for amplifying human HMGCL cDNA

<400> 17

ggtacgtctc ctgtgctctt g 21

<210> 18

<211> 22

<212> DNA

<213> Artificial Sequence

<220>

<223> Oligonucleotide reverse primer used for amplifying human HMGCL cDNA

<400> 18

gtggagattt tccagagatg ct 22

<210> 19

<211> 23

<212> DNA

<213> Artificial Sequence

<220>

<223> Oligonucleotide forward primer used for amplifying human EST(AI051454) cDNA

<400> 19

ggtgggtgtt agttagctcc tct 23

<210> 20

<211> 23

<212> DNA

<213> Artificial Sequence

<220>

<223> Oligonucleotide reverse primer used for amplifying human EST(AI05 1454) cDNA

<400> 20

cttagagcca ccaaagatgg ata 33

<210> 21

<211> 23

<212> DNA

<213> Artificial Sequence

<220>

<223> Oligonucleotide forward primer used for amplifying human KLF4 cDN A

<400> 21

gtcaagttcc caactgagtc atc 23

<210> 22

<211> 23

<212> DNA

<213> Artificial Sequence

<220>

<223> Oligonucleotide reverse primer used for amplifying human KLF4 cDN A

<400> 22

tgaaaccaca gtgcataaca gac 23

<210> 23

<211> 23

<212> DNA

<213> Artificial Sequence

<220>

<223> Oligonucleotide forward primer used for amplifying human H3F3A cDNA

<400> 23

ctcgcgtaag taaggaggtc tct 23

<210> 24

<211> 23

<212> DNA

<213> Artificial Sequence

<220>

<223> Oligonucleotide reverse primer used for amplifying human H3F3A cDNA

<400> 24

actgccaata cctgatttct gtc 23

<210> 25

<211> 23

<212> DNA

<213> Artificial Sequence

<220>

<223> Oligonucleotide forward primer used for amplifying human ACTB cDNA

<400> 25

catccacgaa actaccttca act 23

<210> 26

<211> 23

<212> DNA

<213> Artificial Sequence

<220>

<223> Oligonucleotide reverse primer used for amplifying human ACTB cDN
A

<400> 26

tctccttaga gagaagtggg gtg 23

<210> 27

<211> 23

<212> DNA

<213> Artificial Sequence

<220>

<223> Oligonucleotide forward primer used for amplifying human DKFZP566
D193 cDNA

<400> 27

cagtacctcc atgagaatgt tcc 23

<210> 28

<211> 23

<212> DNA

<213> Artificial Sequence

<220>

<223> Oligonucleotide reverse primer used for amplifying human DKFZP566

D193 cDNA

<400> 28

gggagagaac atgaaagaca ctg 23

<210> 29

<211> 23

<212> DNA

<213> Artificial Sequence

<220>

<223> Oligonucleotide forward primer used for amplifying human APEX cDNA

<400> 29

ccttcaagag accaaatgtt cag 23

<210> 30

<211> 23

<212> DNA

<213> Artificial Sequence

<220>

<223> Oligonucleotide reverse primer used for amplifying human APEX cDNA

<400> 30

ctggttggag gattcctagt ttt 23

<210> 31

<211> 22

<212> DNA

<213> Artificial Sequence

<220>

<223> Oligonucleotide forward primer used for amplifying human DRIL1 cDNA

<400> 31

ataaaacagg ggaaaaccaa gg 22

<210> 32

<211> 22

<212> DNA

<213> Artificial Sequence

<220>

<223> Oligonucleotide reverse primer used for amplifying human DRIL1 cDNA

<400> 32

agtttccctg aaggaacaca aa 22

<210> 33

<211> 21

<212> DNA

<213> Artificial Sequence

<220>

<223> Oligonucleotide forward primer used for amplifying human BIN1 cDNA

<400> 33

cctgtttgag gacacgtttg t 21

<210> 34

<211> 22

<212> DNA

<213> Artificial Sequence

<220>

<223> Oligonucleotide reverse primer used for amplifying human BIN1 cDNA

<400> 34

attttcaaac agcacacaga cc 22

<210> 35

<211> 23

<212> DNA

<213> Artificial Sequence

<220>

<223> Oligonucleotide forward primer used for amplifying human EST(AA495984) cDNA

<400> 35

agacctacaa gatgtgggaa tca 23

<210> 36

<211> 23

<212> DNA

<213> Artificial Sequence

<220>

<223> Oligonucleotide reverse primer used for amplifying human EST(AA495984) cDNA

<400> 36

gagatggtat gaacagggac tga 23

<210> 37

<211> 22

<212> DNA

<213> Artificial Sequence

<220>

<223> Oligonucleotide forward primer used for amplifying human CLTH cDN
A

<400> 37

ggcaagtcca taggtctgaa tc 22

<210> 38

<211> 21

<212> DNA

<213> Artificial Sequence

<220>

<223> Oligonucleotide reverse primer used for amplifying human CLTH cDN
A

<400> 38

gaactgtcct tcttaaggcc c 21

<210> 39

<211> 22

<212> DNA

<213> Artificial Sequence

<220>

<223> Oligonucleotide forward primer used for amplifying human M6PR cDN
A

<400> 39

gtgatggtac cgtataatcc tg 22

<210> 40

<211> 23

<212> DNA

<213> Artificial Sequence

<220>

<223> Oligonucleotide reverse primer used for amplifying human M6PR cDNA

<400> 40

ttctgtaccc tatagtagcc tcc 23

<210> 41

<211> 23

<212> DNA

<213> Artificial Sequence

<220>

<223> Oligonucleotide forward primer used for amplifying human KIAA0106 cDNA

<400> 41

gtcatcacag ccaaggtttt tag 23

<210> 42

<211> 23

<212> DNA

<213> Artificial Sequence

<220>

<223> Oligonucleotide reverse primer used for amplifying human KIAA0106

cDNA

<400> 42

tctagaaaat agcaacccac tgc 23

<210> 43

<211> 22

<212> DNA

<213> Artificial Sequence

<220>

<223> Oligonucleotide forward primer used for amplifying human IGF2R cDNA

<400> 43

tagcatttta attctctccc cc 22

<210> 44

<211> 22

<212> DNA

<213> Artificial Sequence

<220>

<223> Oligonucleotide reverse primer used for amplifying human IGF2R cDNA

<400> 44

tgttgaaatc aaacaagcca gc 22

<210> 45

<211> 23

<212> DNA

<213> Artificial Sequence

<220>

<223> Oligonucleotide forward primer used for amplifying human IDH1 cDNA

<400> 45

tagtaccagc ctaggaattc ggt 23

<210> 46

<211> 23

<212> DNA

<213> Artificial Sequence

<220>

<223> Oligonucleotide reverse primer used for amplifying human IDH1 cDNA

<400> 46

aataaaacag gccagcagaa gtc 23

<210> 47

<211> 23

<212> DNA

<213> Artificial Sequence

<220>

<223> Oligonucleotide forward primer used for amplifying human EST(AI333449) cDNA

<400> 47

cattcctttc cctttatgac agc 23

<210> 48

<211> 23

<212> DNA

<213> Artificial Sequence

<220>

<223> Oligonucleotide reverse primer used for amplifying human EST(AI333449) cDNA

<400> 48

tacaaccttt tccttcacct ctg 23

<210> 49

<211> 21

<212> DNA

<213> Artificial Sequence

<220>

<223> Oligonucleotide forward primer used for amplifying human SDHB cDNA

<400> 49

cacagctccc cgtatcaaga a 21

<210> 50

<211> 23

<212> DNA

<213> Artificial Sequence

<220>

<223> Oligonucleotide reverse primer used for amplifying human SDHB cDNA

<400> 50

ggtatagaga gaatgggtcc tgc 23

<210> 51

<211> 21

<212> DNA

<213> Artificial Sequence

<220>

<223> Oligonucleotide forward primer used for amplifying human TNRC3 cD NA

<400> 51

cccctccaaa agtcttgata g 21

<210> 52

<211> 21

<212> DNA

<213> Artificial Sequence

<220>

<223> Oligonucleotide reverse primer used for amplifying human TNRC3 cD NA

<400> 52

atccaaggag tacagaccag tag 23

<210> 53

<211> 22

<212> DNA

<213> Artificial Sequence

<220>

<223> Oligonucleotide forward primer used for amplifying human MGP cDNA

<400> 53

aagagaaggt taccagtgcc ag 22

<210> 54

<211> 23

<212> DNA

<213> Artificial Sequence

<220>

<223> Oligonucleotide reverse primer used for amplifying human MGP cDNA

<400> 54

aggaaattac aggtttcctc cac 23

<210> 55

<211> 25

<212> DNA

<213> Artificial Sequence

<220>

<223> Oligonucleotide forward primer used for amplifying human CBLB cDNA

<400> 55

gtcaaaacta acagaacatc acagg 25

<210> 56

<211> 22

<212> DNA

<213> Artificial Sequence

<220>

<223> Oligonucleotide reverse primer used for amplifying human CBLB cDNA

<400> 56

aaaacccctt acaaaaggca gt 22

<210> 57

<211> 23

<212> DNA

<213> Artificial Sequence

<220>

<223> Oligonucleotide forward primer used for amplifying human EST(AA055355) cDNA

<400> 57

aaacagctaa agcttcactg cac 23

<210> 58

<211> 23

<212> DNA

<213> Artificial Sequence

<220>

<223> Oligonucleotide reverse primer used for amplifying human EST(AA055355) cDNA

<400> 58

agcaacaggg tagacagttc aag 23

<210> 59

<211> 23

<212> DNA

<213> Artificial Sequence

<220>

<223> Oligonucleotide forward primer used for amplifying human FLJ10803

cDNA

<400> 59

tctaccctgt acgtcagttg gat 23

<210> 60

<211> 23

<212> DNA

<213> Artificial Sequence

<220>

<223> Oligonucleotide reverse primer used for amplifying human FLJ10803 cDNA

<400> 60

caggagtgtc tgtcactgag atg 23

<210> 61

<211> 23

<212> DNA

<213> Artificial Sequence

<220>

<223> Oligonucleotide forward primer used for amplifying human IMPDH1 c DNA

<400> 61

ctggcgagta cttcttctca gac 23

<210> 62

<211> 22

<212> DNA

<213> Artificial Sequence

<220>

<223> Oligonucleotide reverse primer used for amplifying human IMPDH1 c DNA

<400> 62

tcccaagtgt gcaaagttaa ag 22

<210> 63

<211> 23

<212> DNA

<213> Artificial Sequence

<220>

<223> Oligonucleotide forward primer used for amplifying human FLJ20489 cDNA

<400> 63

cccttcaact agagctttca cct 23

<210> 64

<211> 23

<212> DNA

<213> Artificial Sequence

<220>

<223> Oligonucleotide reverse primer used for amplifying human FLJ20489 cDNA

<400> 64

gtgacagtga cacaaataag gca 23

<210> 65

<211> 23

<212> DNA

<213> Artificial Sequence

<220>

<223> Oligonucleotide forward primer used for amplifying human GTF2I cD NA

<400> 65

gaggctggag aagatactca gaa 23

<210> 66

<211> 23

<212> DNA

<213> Artificial Sequence

<220>

<223> Oligonucleotide reverse primer used for amplifying human GTF2I cD NA

<400> 66

gctgagatgt tccagtagga gaa 23

<210> 67

<211> 22

<212> DNA

<213> Artificial Sequence

<220>

<223> Oligonucleotide forward primer used for amplifying human CGI-57 c DNA

<400> 67

cccactcttc ttcataactg cc 22

<210> 68

<211> 23

<212> DNA

<213> Artificial Sequence

<220>

<223> Oligonucleotide reverse primer used for amplifying human CGI-57 cDNA

<400> 68

cctaggagca ggacactaag aga 23

<210> 69

<211> 23

<212> DNA

<213> Artificial Sequence

<220>

<223> Oligonucleotide forward primer used for amplifying human LOC51312 cDNA

<400> 69

actggcttgt ttctctttat ggc 23

<210> 70

<211> 23

<212> DNA

<213> Artificial Sequence

<220>

<223> Oligonucleotide reverse primer used for amplifying human LOC51312 cDNA

<400> 70

ccacagtgaa gcagaaaagc tac 23

<210> 71

<211> 23

<212> DNA

<213> Artificial Sequence

<220>

<223> Oligonucleotide forward primer used for amplifying human CHAC cDN A

<400> 71

agccttaaca atgcctactt tcc 23

<210> 72

<211> 23

<212> DNA

<213> Artificial Sequence

<220>

<223> Oligonucleotide reverse primer used for amplifying human CHAC cDN A

<400> 72

aaggagcctg agaagaattt cac 23

<210> 73

<211> 25

<212> DNA

<213> Artificial Sequence

<220>

<223> Oligonucleotide forward primer used for amplifying human ATP1B1 c

DNA

<400> 73

tgggacctac acttaatcta tatgc 25

<210> 74

<211> 23

<212> DNA

<213> Artificial Sequence

<220>

<223> Oligonucleotide reverse primer used for amplifying human ATP1B1 c
DNA

<400> 74

gtccgttgtt ttaacctgac tga 23

<210> 75

<211> 23

<212> DNA

<213> Artificial Sequence

<220>

<223> Oligonucleotide forward primer used for amplifying human CPT1A cD
NA

<400> 75

cagctagatc atgcactgtt gac 23

<210> 76

<211> 23

<212> DNA

<213> Artificial Sequence

<220>

<223> Oligonucleotide reverse primer used for amplifying human CPT1A cDNA

<400> 76

gcatactgta tttacatgca ccg 23

<210> 77

<211> 22

<212> DNA

<213> Artificial Sequence

<220>

<223> Oligonucleotide forward primer used for amplifying human CTSG cDNA

<400> 77

cttctgctgg cctttctcct ac 22

<210> 78

<211> 23

<212> DNA

<213> Artificial Sequence

<220>

<223> Oligonucleotide reverse primer used for amplifying human CTSG cDNA

<400> 78

tgtggacgtt tattaaggct ctg 23

<210> 79

<211> 23

<212> DNA

<213> Artificial Sequence

<220>

<223> Oligonucleotide forward primer used for amplifying human AXUD1 cD NA

<400> 79

ctaggtgtgg atttgagctc tgt 23

<210> 80

<211> 23

<212> DNA

<213> Artificial Sequence

<220>

<223> Oligonucleotide reverse primer used for amplifying human AXUD1 cD NA

<400> 80

cctgtaagct caggtacaag gtg 23

<210> 81

<211> 23

<212> DNA

<213> Artificial Sequence

<220>

<223> Oligonucleotide forward primer used for amplifying human HLA-B cD NA

<400> 81

ctaggtgtgg atttgagctc tgt 23

<210> 82

<211> 23

<212> DNA

<213> Artificial Sequence

<220>

<223> Oligonucleotide reverse primer used for amplifying human HLA-B cDNA

<400> 82

cctgtaagct caggtacaag gtg 23

<210> 83

<211> 23

<212> DNA

<213> Artificial Sequence

<220>

<223> Oligonucleotide forward primer used for amplifying human GOLGA4 cDNA

<400> 83

aattatggag ctacagacac agc 23

<210> 84

<211> 27

<212> DNA

<213> Artificial Sequence

<220>

<223> Oligonucleotide reverse primer used for amplifying human GOLGA4 cDNA

<400> 84

ttatctgttt ctcagagatg acactgc 27

<210> 85

<211> 23

<212> DNA

<213> Artificial Sequence

<220>

<223> Oligonucleotide forward primer used for amplifying human EST(AA74 3462) cDNA

<400> 85

cagcagtgcc ttatatagtt gcc 23

<210> 86

<211> 23

<212> DNA

<213> Artificial Sequence

<220>

<223> Oligonucleotide reverse primer used for amplifying human EST(AA74 3462) cDNA

<400> 86

gcacatggtt cactgaagat aca 23

<210> 87

<211> 23

<212> DNA

<213> Artificial Sequence

<220>

<223> Oligonucleotide forward primer used for amplifying human SCYA13 c

DNA

<400> 87

acctcaaca tgaaagtctc tgc 23

<210> 88

<211> 23

<212> DNA

<213> Artificial Sequence

<220>

<223> Oligonucleotide reverse primer used for amplifying human SCYA13 c DNA

<400> 88

cacaaagagc tctccttcct aca 23

<210> 89

<211> 21

<212> DNA

<213> Artificial Sequence

<220>

<223> Oligonucleotide forward primer used for amplifying human B4GALT1 cDNA

<400> 89

ctaatcccag ggttgagcct t 21

<210> 90

<211> 23

<212> DNA

<213> Artificial Sequence

<220>

<223> Oligonucleotide reverse primer used for amplifying human B4GALT1 cDNA

<400> 90

ttgaacacca agatcagaca cag 23

<210> 91

<211> 23

<212> DNA

<213> Artificial Sequence

<220>

<223> Oligonucleotide forward primer used for amplifying human DKFZP564 00463 cDNA

<400> 91

ttctgaccgg caggtatatt atg 23

<210> 92

<211> 23

<212> DNA

<213> Artificial Sequence

<220>

<223> Oligonucleotide reverse primer used for amplifying human DKFZP564 00463 cDNA

<400> 92

acaactgcca ctacgtgttt ctt 23

<210> 93

<211> 21

<212> DNA

<213> Artificial Sequence

<220>

<223> Oligonucleotide forward primer used for amplifying human HSJ1 cDN A

<400> 93

ctaccacaaa tcagggctca g 21

<210> 94

<211> 22

<212> DNA

<213> Artificial Sequence

<220>

<223> Oligonucleotide reverse primer used for amplifying human HSJ1 cDN A

<400> 94

attgacttcc agcatttctt cc 22

<210> 95

<211> 22

<212> DNA

<213> Artificial Sequence

<220>

<223> Oligonucleotide forward primer used for amplifying human MRPL3 cD NA

<400> 95

cgccgaaaca gacagttaaa at 22

<210> 96

<211> 21

<212> DNA

<213> Artificial Sequence

<220>

<223> Oligonucleotide reverse primer used for amplifying human MRPL3 cDNA

<400> 96

cctttgtccg gcctttaaca t 21

<210> 97

<211> 23

<212> DNA

<213> Artificial Sequence

<220>

<223> Oligonucleotide forward primer used for amplifying human C9orf10 cDNA

<400> 97

tcgtgactaa taccatgcat ctg 23

<210> 98

<211> 23

<212> DNA

<213> Artificial Sequence

<220>

<223> Oligonucleotide reverse primer used for amplifying human C9orf10 cDNA

<400> 98

ttcagataca ccacaccaca atc 23

<210> 99

<211> 23

<212> DNA

<213> Artificial Sequence

<220>

<223> Oligonucleotide forward primer used for amplifying human DDX1 cDNA

<400> 99

ccagagatgt ggtctgaagc tat 23

<210> 100

<211> 25

<212> DNA

<213> Artificial Sequence

<220>

<223> Oligonucleotide reverse primer used for amplifying human DDX1 cDNA

<400> 100

cgttactata aatactgctg cttgg 25

<210> 101

<211> 22

<212> DNA

<213> Artificial Sequence

<220>

<223> Oligonucleotide forward primer used for amplifying human EST(AA42

1326) cDNA

<400> 101

gagagccctt aagtctgtcc tg 22

<210> 102

<211> 23

<212> DNA

<213> Artificial Sequence

<220>

<223> Oligonucleotide reverse primer used for amplifying human EST(AA42 1326) cDNA

<400> 102

gaggcttcag ttctgtacca ttt 23

<210> 103

<211> 22

<212> DNA

<213> Artificial Sequence

<220>

<223> Oligonucleotide forward primer used for amplifying human HLA-A cD NA

<400> 103

cagtattggg agtggaccac ag 22

<210> 104

<211> 23

<212> DNA

<213> Artificial Sequence

<220>

<223> Oligonucleotide reverse primer used for amplifying human HLA-A cDNA

<400> 104

tatgtatgtt cgtgaggcac aag 23

<210> 105

<211> 23

<212> DNA

<213> Artificial Sequence

<220>

<223> Oligonucleotide forward primer used for amplifying human STIM1 cDNA

<400> 105

aaggagcctc atcctaatct cac 23

<210> 106

<211> 22

<212> DNA

<213> Artificial Sequence

<220>

<223> Oligonucleotide reverse primer used for amplifying human STIM1 cDNA

<400> 106

aacacagaaa accccatttg ag 22

<210> 107

<211> 23

<212> DNA

<213> Artificial Sequence

<220>

<223> Oligonucleotide forward primer used for amplifying human AMPD2 cD NA

<400> 107

actataccaa ggaaggccct gag 23

<210> 108

<211> 23

<212> DNA

<213> Artificial Sequence

<220>

<223> Oligonucleotide reverse primer used for amplifying human AMPD2 cD NA

<400> 108

tctcatgcac taggaaagat ggt 23

<210> 109

<211> 21

<212> DNA

<213> Artificial Sequence

<220>

<223> Oligonucleotide forward primer used for amplifying human STX5A cD NA

<400> 109

agagaacctg aagcagcaga g 21

<210> 110

<211> 23

<212> DNA

<213> Artificial Sequence

<220>

<223> Oligonucleotide reverse primer used for amplifying human STX5A cDNA

<400> 110

gaggctgaca gtcttgctgt aat 23

<210> 111

<211> 23

<212> DNA

<213> Artificial Sequence

<220>

<223> Oligonucleotide forward primer used for amplifying human IFNB1 cDNA

<400> 111

ctttccatga gctacaactt gct 23

<210> 112

<211> 23

<212> DNA

<213> Artificial Sequence

<220>

<223> Oligonucleotide reverse primer used for amplifying human IFNB1 cDNA

<400> 112

tcagtttcgg aggtaacctg taa 23

<210> 113

<211> 23

<212> DNA

<213> Artificial Sequence

<220>

<223> Oligonucleotide forward primer used for amplifying human MAEA cDNA

<400> 113

atcaccgagg gaaacttaag aac 23

<210> 114

<211> 22

<212> DNA

<213> Artificial Sequence

<220>

<223> Oligonucleotide reverse primer used for amplifying human MAEA cDNA

<400> 114

gctagtcaac atcaacatgc aa 22

<210> 115

<211> 23

<212> DNA

<213> Artificial Sequence

<220>

<223> Oligonucleotide forward primer used for amplifying human LBR cDNA

<400> 115

taccaaacct gactccctgt aaa 23

<210> 116

<211> 23

<212> DNA

<213> Artificial Sequence

<220>

<223> Oligonucleotide reverse primer used for amplifying human LBR cDNA

<400> 116

tgtttaaaga ctaccggatg ctg 23

<210> 117

<211> 23

<212> DNA

<213> Artificial Sequence

<220>

<223> Oligonucleotide forward primer used for amplifying human LIMK2 cD
NA

<400> 117

tgtttacctg ctcactggct cta 23

<210> 118

<211> 23

<212> DNA

<213> Artificial Sequence

<220>

<223> Oligonucleotide reverse primer used for amplifying human LIMK2 cD

NA

<400> 118

tagtctgatc aatggcccag ttc 23

<210> 119

<211> 22

<212> DNA

<213> Artificial Sequence

<220>

<223> Oligonucleotide forward primer used for amplifying human EST(AI365683) cDNA

<400> 119

cagctccaaa accaacctag tc 22

<210> 120

<211> 22

<212> DNA

<213> Artificial Sequence

<220>

<223> Oligonucleotide reverse primer used for amplifying human EST(AI365683) cDNA

<400> 120

ataaccactc tttcctgccc tg 22

<210> 121

<211> 23

<212> DNA

<213> Artificial Sequence

<220>

<223> Oligonucleotide forward primer used for amplifying human RPL26 cDNA

<400> 121

aggttttcag cacaagtcac act 23

<210> 122

<211> 23

<212> DNA

<213> Artificial Sequence

<220>

<223> Oligonucleotide reverse primer used for amplifying human RPL26 cDNA

<400> 122

gacagcctca cttctgtatg gac 23

<210> 123

<211> 21

<212> DNA

<213> Artificial Sequence

<220>

<223> Oligonucleotide forward primer used for amplifying human FLJ10209 cDNA

<400> 123

accatacatg ctgtctgtgg c 21

<210> 124

<211> 21

<212> DNA

<213> Artificial Sequence

<220>

<223> Oligonucleotide reverse primer used for amplifying human FLJ10209 cDNA

<400> 124

aacatcaact cagtccctcc c 21

<210> 125

<211> 22

<212> DNA

<213> Artificial Sequence

<220>

<223> Oligonucleotide forward primer used for amplifying human FAAH cDN A

<400> 125

gtaaccaaaa ccctcactgc tc 22

<210> 126

<211> 23

<212> DNA

<213> Artificial Sequence

<220>

<223> Oligonucleotide reverse primer used for amplifying human FAAH cDN A

<400> 126

gtccgtcaaa ctgcttctct tta 23

<210> 127

<211> 21

<212> DNA

<213> Artificial Sequence

<220>

<223> Oligonucleotide forward primer used for amplifying human C21ORF33 cDNA

<400> 127

gtgtcctgaa ggagttccac c 21

<210> 128

<211> 23

<212> DNA

<213> Artificial Sequence

<220>

<223> Oligonucleotide reverse primer used for amplifying human C21ORF33 cDNA

<400> 128

acaaagtcta acgccaacac act 23

<210> 129

<211> 23

<212> DNA

<213> Artificial Sequence

<220>

<223> Oligonucleotide forward primer used for amplifying human EST(Z44513) cDNA

<400> 129

catgtactga ggttgagtcg tga 23

<210> 130

<211> 23

<212> DNA

<213> Artificial Sequence

<220>

<223> Oligonucleotide reverse primer used for amplifying human EST(Z445 13) cDNA

<400> 130

cagcttttca cacaacagtc aag 23

<210> 131

<211> 21

<212> DNA

<213> Artificial Sequence

<220>

<223> Oligonucleotide forward primer used for amplifying human PRKACA c DNA

<400> 131

aagaggggct cacgcttaac t 21

<210> 132

<211> 23

<212> DNA

<213> Artificial Sequence

<220>

<223> Oligonucleotide reverse primer used for amplifying human PRKACA c

DNA

<400> 132

gcacactgtt caagaggaag tct 23

<210> 133

<211> 23

<212> DNA

<213> Artificial Sequence

<220>

<223> Oligonucleotide forward primer used for amplifying human EPB49 cDNA

<400> 133

gaagaaggcc tctctcttct gat 23

<210> 134

<211> 23

<212> DNA

<213> Artificial Sequence

<220>

<223> Oligonucleotide reverse primer used for amplifying human EPB49 cDNA

<400> 134

cttttaattc caagcagagt ccc 23

<210> 135

<211> 23

<212> DNA

<213> Artificial Sequence

<220>

<223> Oligonucleotide forward primer used for amplifying human EST(U51712) cDNA

<400> 135

cagatccgtc acagactaag gag 23

<210> 136

<211> 22

<212> DNA

<213> Artificial Sequence

<220>

<223> Oligonucleotide reverse primer used for amplifying human EST(U51712) cDNA

<400> 136

gttggagttt ctgtcttctg gc 22

<210> 137

<211> 22

<212> DNA

<213> Artificial Sequence

<220>

<223> Oligonucleotide forward primer used for amplifying human CRSP9 cDNA

<400> 137

gcaatcagtt ccaatgtgat ga 22

<210> 138

<211> 23

<212> DNA

<213> Artificial Sequence

<220>

<223> Oligonucleotide reverse primer used for amplifying human CRSP9 cDNA

<400> 138

tacaacagca gctaacactt tgc 23

<210> 139

<211> 22

<212> DNA

<213> Artificial Sequence

<220>

<223> Oligonucleotide forward primer used for amplifying human EST(AA600323) cDNA

<400> 139

ctctcaccag caggctaatt tt 22

<210> 140

<211> 23

<212> DNA

<213> Artificial Sequence

<220>

<223> Oligonucleotide reverse primer used for amplifying human EST(AA600323) cDNA

<400> 140

caattaaggg gaaacagtga gtg 23

<210> 141

<211> 21

<212> DNA

<213> Artificial Sequence

<220>

<223> Oligonucleotide forward primer used for amplifying human STK22B c
DNA

<400> 141

acatcctggc aactgtcaac c 21

<210> 142

<211> 22

<212> DNA

<213> Artificial Sequence

<220>

<223> Oligonucleotide reverse primer used for amplifying human STK22B c
DNA

<400> 142

attttacttg cacctgtgcc tt 22

<210> 143

<211> 23

<212> DNA

<213> Artificial Sequence

<220>

<223> Oligonucleotide forward primer used for amplifying human TRB@ cDN
A

<400> 143

ctcaatgact ccagatactg cct 23

<210> 144

<211> 23

<212> DNA

<213> Artificial Sequence

<220>

<223> Oligonucleotide reverse primer used for amplifying human TRB@ cDNA

<400> 144

tagcctattt cgtacttggg gtt 23

<210> 145

<211> 23

<212> DNA

<213> Artificial Sequence

<220>

<223> Oligonucleotide forward primer used for amplifying human EEF1D cDNA

<400> 145

tggctacaaa cttcctagca cat 23

<210> 146

<211> 23

<212> DNA

<213> Artificial Sequence

<220>

<223> Oligonucleotide reverse primer used for amplifying human EEF1D cD

NA

<400> 146

ctccaccaca cactgaatct gta 23

<210> 147

<211> 22

<212> DNA

<213> Artificial Sequence

<220>

<223> Oligonucleotide forward primer used for amplifying human RPGR cDNA

<400> 147

gtcacaaggg attttcatga cg 22

<210> 148

<211> 23

<212> DNA

<213> Artificial Sequence

<220>

<223> Oligonucleotide reverse primer used for amplifying human RPGR cDNA

<400> 148

gcaatactga acagttaagg cca 23

<210> 149

<211> 23

<212> DNA

<213> Artificial Sequence

<220>

<223> Oligonucleotide forward primer used for amplifying human ARRB1 cDNA

<400> 149

gctcaagaca cgagagaaga tgt 23

<210> 150

<211> 23

<212> DNA

<213> Artificial Sequence

<220>

<223> Oligonucleotide reverse primer used for amplifying human ARRB1 cDNA

<400> 150

ctactcctgg ggtctacgaa ctt 23

<210> 151

<211> 22

<212> DNA

<213> Artificial Sequence

<220>

<223> Oligonucleotide forward primer used for amplifying human NOP5/NOP58 cDNA

<400> 151

atccttctgg tgactccaca ct 22

<210> 152

<211> 22

<212> DNA

<213> Artificial Sequence

<220>

<223> Oligonucleotide reverse primer used for amplifying human NOP5/NOP58 cDNA

<400> 152

ctgtgctggt acatggttct tc 22

<210> 153

<211> 23

<212> DNA

<213> Artificial Sequence

<220>

<223> Oligonucleotide forward primer used for amplifying human IGL@ cDNA

<400> 153

tgatttatga ggtcagtaag cgg 23

<210> 154

<211> 23

<212> DNA

<213> Artificial Sequence

<220>

<223> Oligonucleotide reverse primer used for amplifying human IGL@ cDNA

<400> 154

gggtgagggt ttagaaccta tga 23

## Description

### Technical Field

The present invention relates to a method for judging sensitivity to imatinib or a derivative thereof or a pharmaceutically acceptable salt thereof. The method of the present invention is useful for judging therapeutic effect of imatiaib or a derivative thereof or a pharmaceutically acceptable salt thereof against, for example, chronic myeloid leukemia (CML) or the like.

### Background Art

CML is a clonal disorder arising from neoplastic transformation of hematopoietic stem cells, most of which are characterized by the presence of a Philadelphia chromosome (Ph) and by constitutive activation of BCR-ABL tyrosine kinase (S. Faderl et al., N Engl J Med 341, 164-72. (1999)). CML progresses through three phases; chronic phase, accelerated phase and invariably fetal blast crisis. Conventional therapeutic options include interferon-α and allogenic stem-cell transplantation (SCT). Interferon-α prolongs overall survival but has considerable adverse effects. SCT is the only curative treatment, but is associated with substantial morbidity and is limited to patients with suitable donors. Thus, the prognosis of CML is still poor.

Development of the ABL-selective tyrosine kinase inhibitor imatinib (4-(4-methylpiperazin-1-ylmethyl)-N-[4-methyl-3-(4-pyridin-3-ylpyrimidin-2-ylamino)phenyl]benzamide), development code name: STI571) was an important advance in the management of CML (E. Buchdunger, A. Matter, B. J. Druker, Biochim Biophys Acta 1551, M11-8. (2001); B. J. Druker et al., Nat Med 2, 561-6. (1996)). With this drug around 90% of CML patients are induced into hematological complete remission, and in more than 60% of patients Ph chromosome-positive leukemia cells are completely or partially reduced without severe adverse effects (B. J. Druker et al., N Engl J Med 344, 1031-7. (2001)). Thus, imatinib has become the first choice drug for the treatment of CML.

Imatinib is an anti-cancer drug having the chemical structure shown by Formula [I] below. Imatinib is widely used for therapy of CML, and besides, it has been reported that it is useful for therapies of other tumors such as gastrointestinal stromal tumor (GSIT). Imatinib mesilate is commercially available from Novartis Pharmaceuticals, Basel, Switzerland under the trademark "Glivec", and is clinically used for therapy of CML.

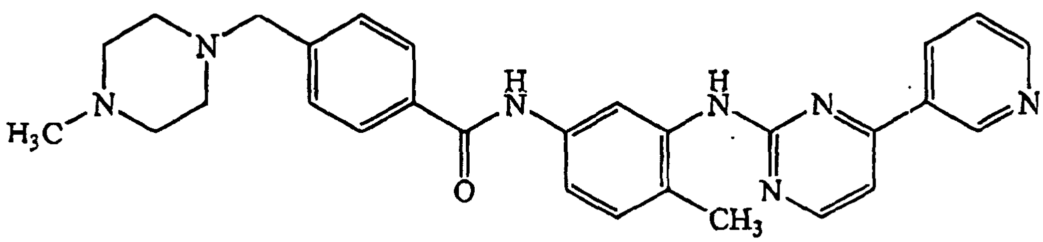

[I]

However, imatinib is effective for not all of the CML patients, and there are patients to whom imatinib is not effective. Since the therapeutic effect of imatinib is prominent when it is effective, it has become difficult to timely decide whether SCT should be performed or not (J. M. Goldman, B. J. Druker, Blood 98, 2039-42. (2001)). To administer imatinib to a patient to whom imatinib is ineffective is waste of time and medical cost, and involves a risk that the patient may lose the chance to receive another therapy. Therefore, if it can be predicted whether the administration of imatinib is effective or not, it is very advantageous to the therapy of CML.

### Disclosure of the Invention

An object of the present invention is to provide a method for judging whether a patient is sensitive to imatinib or not, in case where the patient is suffering from a disease to be treated by administration of imatinib, that is, to provide a method for predicting whether administration of imatinib to the patient is effective for the therapy of the disease or not.

The present inventors inferred that expression amounts of specific genes may be different between the patients having sensitivity to imatinib, that is, responders to whom administration of imatinib is effective, and the patients who do not have sensitivity to imatinib, that is, non-responders to whom administration of imatinib is ineffective. Thus, the inventors measured expression amounts of various genes in mononuclear cells of CML patients using cDNA microarrays on which not less than 20,000 types of cDNAs were immobilized, and checked whether there were genes whose expression amounts are statistically different between responders and non-responders. As a result, the inventors discovered that there were significant differences in the expression amounts of 77 types of genes. Further, the inventors experimentally confirmed that it can be predicted whether a new patient not involved in the above-mentioned statistical processing is a responder or non-responder based on the expression amounts of the genes, thereby completing the present invention.

That is, the present invention provides a method of judging sensitivity to imatinib or a derivative thereof or a pharmaceutically acceptable salt thereof, comprising measuring expression amounts of a plurality of genes selected from the group consisting of the following genes (1) to (77) in sample cells separated from body; and comparing the measured amounts with those of responders and non-responders to imatinib or a derivative thereof or a pharmaceutically acceptable salt thereof:

(1)HN1(AI086871), (2)AKR1C3(D17793), (3)QARS(X76013),
(4)KIAA1105(AA136180), (5)KIAA0668(AA506972), (6)BLCAP(AF053470),
(7)ADFP(X97324), (8)FLJ10422(AA894857), (9)HMGCL(L07033),
(10)EST(AI051454), (11)KLF4(AI290876), (12)H3F3A(M11354),
(13)ACTB(V00478), (14)DKFZP566D193(AA401318), (15)APEX(U79268),
(16)DRIL1(U88047), (17)BIN1(AF001383), (18)EST(AA495984),
(19)CLTH(N41902), (20)M6PR(AA179832), (21)KIAA0106(D14662),
(22)IGF2R(J03528), (23)IDH1(AA330014), (24)EST(AI333449),
(25)SDHB(AA365986), (26)TNRC3(AI743134), (27)MGP(AA156488),
(28)CBLB(U26710), (29)EST(AA055355), (30)FLJ10803(T70782),
(31)IMPDH1(J05272), (32)FLJ20489(AI091459), (33)GTF2I(U77948), (34)CGI-57(AF070638), (35)LOC51312(. AI128538), (36)CHAC(AA228874),
(37)ATPIB1(X03747), (38)CPTIA(AA632225), (39)CTSG(M1G117),
(40)AXUD1(AI091372), (41)HLA-B(M28204), (42)GOLGA4(U31906),
(43)EST(AA743462), (44)SCYA13(U46767), (45)B4GALT1(D29805),
(46)DKFZP564O0463(AA143048), (47)HSJ1(X63368), (48)MRPL3(X06323),
(49)C9orf10(D80005), (50)DDX1(X70649), (51)EST(AA421326), (52)HLA-A(AF055066), (53)STIM1(AA101834), (54)AMPD2(M91029),
(55)STX5A(U26648), (56)IFNB1(M25460), (57)MAEA(AI291745),
(58)LBR(L25941), (59)LTMK2(D45906), (60)EST(AI365683),
(61)RPL26(AA778161), (62)FLJ10209(AL137271), (63)FAAH(AA132519),
(64)C21ORF33(Y07572), (65)EST(Z44513), (66)PRKACA(X07767),
(67)EPB49(L19713), (68)EST(U51712), (69)CRSP9(AI334396),
(70)EST(AA600323), (71)STK22B(L77564), (72)TRB@(X01410),
(73)EEF1D(Z21507), (74)RPGR(U57629), (75)ARRB1(AA918725),
(76)NOP5/NOP58(AA602490), (77)IGL@(M87790) (wherein the characters in parentheses after the symbol of each gene denote GenBank Accession No.)

By the present invention, it may be predicted whether administration of imatinib to a patient is effective for the therapy of the disease or not. Therefore, waste of time and medical cost incurred by administering imatinib to a patient to whom administration of imatinib is not effective may be prevented, and the risk that the patient loses a chance to receive another therapy may be decreased.

Brief Description of the Drawings

Fig. 1 shows the relationship between the number of discriminating genes and the classification score (CS).

Fig. 2 shows the prediction score obtained using varying number of discriminating genes. "R" denotes responders, and "N" denotes non-responders.

Fig. 3 shows the results of clustering analysis using 15 or 30 prediction gene set. All of the samples were classified depending on the sensitivity to imatinib.

Fig. 4 shows prediction score of individual patient. Filled circles and filled triangles indicate scores in cross-validation cases of patients whose expression data were used for selecting discriminating genes (learning). Open circles and open triangles represent scores for four additional (test) cases. Circles indicate CML patients in chronic phase and triangles show CML patients in blast crisis (learning) and accelerated phases (test), respectively. High

absolute values indicate high confidence.

Best Mode for Carrying Out the Invention

The 77 types of genes which may be used in the method of the present invention were selected by judging whether the expression amounts of the respective genes in mononuclear cells of CML patients are statistically significantly ($P<0.05$) different or not between responders and non-responders, by the method which will be described in detail in Example below. The ranks, p-values, symbols, GenBank Accession Nos., and whether the expression amounts in non-responders are larger or smaller than that those in responders (the cases where it is larger are indicated by the symbol " ↑", and the cases where it is smaller are indicated by the symbol "↓") are summarized in Tables 1 to 4 below. The order of listing in the tables is the ascending order of p-value, that is, the descending order of the magnitude of the statistical significant difference. As is apparent from the fact that GenBank Accession Nos, have been assigned to all of these genes, these genes *per se* as well as their nucleotide sequences arc known and registered in GenBank. GenBank is a database presented by a U.S. governmental organization, collecting sequences of genes and proteins, and anybody can access through internet with no charge, so that the sequences of the genes may easily be obtained.

Table 1

| Rank | Permutation P-value | GenBank ID | Symbol | Gene name | Expression in Non-responders |
|---|---|---|---|---|---|
| 1 | 0.0003 | AI086871 | HN1 | Humanin | ↓ |
| 2 | 0.0003 | D17793 | AKR1C3 | aldo-keto reductase family 1, member C3 | ↑ |
| 3 | 0.0013 | X76013 | QARS | glutaminyl-tRNA synthetase | ↓ |
| 4 | 0.0015 | AA136180 | KIAA1105 | KIAA1105 protein | ↓ |
| 5 | 0.0019 | AA506972 | KIAA0668 | KIAA0668 protein | ↓ |
| 6 | 0.0020 | AF053470 | BLCAP | bladder cancer associated protein | ↓ |
| 7 | 0.0021 | X97324 | ADFP | adipose differentiation-related protein | ↑ |
| 8 | 0.0029 | AA894857 | FLJ10422 | hypothetical protein FLJ10422 | ↑ |
| 9 | 0.0038 | L07033 | HMGCL | 3-hydroxymethyl-3-methylglutaryl-Coenzyme A lyase | ↑ |
| 10 | 0.0040 | AI051454 | EST | EST | ↑ |
| 11 | 0.0063 | AI290876 | KLF4 | Kruppel-like factor 4 | ↓ |
| 12 | 0.0083 | M11354 | H3F3A | H3 histone, family 3A | ↓ |
| 13 | 0.0094 | V00478 | ACTB | actin, beta | ↓ |
| 14 | 0.0094 | AA401318 | DKFZP566D193 | DKFZP566D193 protein | ↑ |
| 15 | 0.0101 | U79268 | APEX | APEX nuclease | ↑ |
| 16 | 0.0107 | U88047 | DRIL1 | dead ringer (Drosophila)-like 1 | ↓ |
| 17 | 0.0113 | AF001383 | BIN1 | bridging integrator 1 | ↓ |
| 18 | 0.0114 | AA495984 | EST | EST | ↑ |
| 19 | 0.0123 | N41902 | CLTH | Clathrin assembly lymphoid-myeloid leukemia gene | ↓ |
| 20 | 0.0127 | AA179832 | M6PR | mannose-6-phosphate receptor | ↑ |

Table 2

| Rank | Permutation P-value | GenBank ID | Symbol | Gene name | Expression in Non-responders |
|---|---|---|---|---|---|
| 21 | 0.0133 | D14662 | KIAA0106 | anti-oxidant protein 2 | ↑ |
| 22 | 0.0139 | J03528 | IGF2R | insulin-like growth factor 2 receptor | ↓ |
| 23 | 0.0151 | AA330014 | IDH1 | isocitrate dehydrogenase 1 (NADP+), soluble | ↑ |
| 24 | 0.0154 | AI333449 | EST | EST | ↑ |
| 25 | 0.0156 | AA365986 | SDHB | succinate dehydrogenase complex, subunit B | ↓ |
| 26 | 0.0165 | AI743134 | TNRC3 | trinucleotide repeat containing 3 | ↑ |
| 27 | 0.0171 | AA156488 | MGP | KIAA1008 protein | ↑ |
| 28 | 0.0178 | U26710 | CBLB | Cas-Br-M ectropic retroviral transforming sequence b | ↑ |
| 29 | 0.0187 | AA055355 | EST | EST | ↑ |
| 30 | 0.0191 | T70782 | FLJ10803 | hypothetical protein FLJ10803 | ↓ |
| 31 | 0.0193 | J05272 | IMPDH1 | IMP (inosine monophosphate) dehydrogenase 1 | ↓ |
| 32 | 0.0197 | AI091459 | FLJ20489 | hypothetical protein FLJ20489 | ↑ |
| 33 | 0.0200 | U77948 | GTF2I | general transcription factor II, i | ↓ |
| 34 | 0.0201 | AF070638 | CGI-57 | hypothetical protein | ↓ |
| 35 | 0.0201 | AI128538 | LOC51312 | mitochondrial solute carrier | ↓ |
| 36 | 0.0205 | AA228874 | CHAC | EST | ↑ |
| 37 | 0.0214 | X03747 | ATP1B1 | ATPase, Na+/K+ transporting, beta 1 polypeptide | ↑ |
| 38 | 0.0228 | AA632225 | CPT1A | carnitine palmitoyltransferase I, liver | ↑ |
| 39 | 0.0262 | M16117 | CTSG | cathepsin G | ↑ |
| 40 | 0.0269 | AI091372 | AXUD1 | Homo sapiens mRNA; cDNA DKFZp566F164 | ↓ |
| 41 | 0.0270 | M28204 | HLA-B | major histocompatibility complex, class I, B | ↓ |

Table 3

| Rank | Permutation P-value | GenBank ID | Symbol | Gene name | Expression in Non-responders |
|---|---|---|---|---|---|
| 42 | 0.0271 | U31906 | GOLGA4 | golgi autoantigen, golgin subfamily a, 4 | ↓ |
| 43 | 0.0272 | AA743462 | EST | EST | ↑ |
| 44 | 0.0279 | U46767 | SCYA13 | small inducible cytokine subfamily A, member 13 | ↓ |
| 45 | 0.0281 | D29805 | B4GALT1 | beta 1,4- galactosyltransferase, polypeptide 1 | ↓ |
| 46 | 0.0290 | AA143048 | DKFZP564O0463 | DKFZP564O0463 protein | ↑ |
| 47 | 0.0314 | X63368 | HSJ1 | heat shock protein, neuronal DNAJ-like 1 | ↓ |
| 48 | 0.0315 | X06323 | MRPL3 | mitochondrial ribosomal protein L3 | ↑ |
| 49 | 0.0320 | D80005 | C9orf10 | C9orf10 protein | ↓ |
| 50 | 0.0327 | X70649 | DDX1 | DEAD/H (Asp-Glu-Ala-Asp/His) box polypeptide 1 | ↑ |
| 51 | 0.0350 | AA421326 | EST | Homo sapiens cDNA: FLJ21918 fis, clone HEP04006 | ↑ |
| 52 | 0.0353 | AF055066 | HLA-A | major histocompatibility complex, class I, A | ↓ |
| 53 | 0.0359 | AA101834 | STIM1 | stroma-interacting molecule 1 | ↓ |
| 54 | 0.0359 | M91029 | AMPD2 | adenosine monophosphate deaminase 2 | ↓ |
| 55 | 0.0361 | U26648 | STX5A | syntaxin 5A | ↓ |
| 56 | 0.0366 | M25460 | IFNB1 | interferon, beta 1, fibroblast | ↓ |
| 57 | 0.0370 | AI291745 | MAEA | macrophage erythroblast attacher | ↑ |
| 58 | 0.0372 | L25941 | LBR | lamin B receptor | ↑ |
| 59 | 0.0373 | D45906 | LIMK2 | LIM domain kinase 2 | ↓ |
| 60 | 0.0387 | AI365683 | EST | Homo sapiens PAC clone RP4-751H13 from 7q35-qter | ↓ |
| 61 | 0.0391 | AA778161 | RPL26 | ribosomal protein L26 | ↑ |
| 62 | 0.0395 | AL137271 | FLJ10209 | hypothetical protein FLJ10209 | ↓ |

Table 4

| Rank | Permutation P-value | GenBank ID | Symbol | Gene name | Expression in Non-responders |
|---|---|---|---|---|---|
| 63 | 0.0407 | AA132519 | FAAH | EST | ↓ |
| 64 | 0.0415 | Y07572 | C21ORF33 | ES1 (zebrafish) protein, human homolog of | ↑ |
| 65 | 0.0427 | Z44513 | EST | EST | ↑ |
| 66 | 0.0432 | X07767 | PRKACA | protein kinase, cAMP-dependent, catalytic, alpha | ↓ |
| 67 | 0.0433 | L19713 | EPB49 | erythrocyte membrane protein band 4.9 (dematin) | ↓ |
| 68 | 0.0439 | U51712 | EST | EST | ↓ |
| 69 | 0.0442 | AI334396 | CRSP9 | cofactor required for Sp1 transcriptional activation | ↓ |
| 70 | 0.0442 | AA600323 | EST | EST | ↑ |
| 71 | 0.0442 | L77564 | STK22B | serine/threonine kinase 22B | ↓ |
| 72 | 0.0444 | X01410 | TRB@ | T cell receptor beta locus | ↓ |
| 73 | 0.0446 | Z21507 | EEF1D | eukaryotic translation elongation factor 1 delta | ↓ |
| 74 | 0.0446 | U57629 | RPGR | retinitis pigmentosa GTPase regulator | ↓ |
| 75 | 0.0454 | AA918725 | ARRB1 | arrestin, beta 1 | ↓ |
| 76 | 0.0458 | AA602490 | NOP5/NOP58 | nucleolar protein NOP5/NOP58 | ↑ |
| 77 | 0.0461 | M87790 | IGL@ | immunoglobulin lambda locus | ↓ |

Information was retrieved from Unigene database in NCBI (build#131).

The numbers (1) to (77) described in the original claim 1 at the time of filing the application are the ascending

order of p-values shown in Table 1. A smaller number indicates larger statistical significant difference.

According to the method of the present invention, expression amounts of a plurality of genes in the group of the above-described genes (1) to (77) are measured. It is not true that the larger the number of the genes whose expression amounts are measured, the more accurate the judgment. Thus, it is preferred to measure the expression amounts of 10 to 35 genes from No. (1), in an ascending order, of the genes (1) to (35). More preferably, the expression amounts of the genes (1) to (15), or (1) to (30), are measured.

As the sample cells separated from the body, the cells presenting the diseased state of the disease to be treated by administration of imatinib are preferred. For example, in case of CML, leukocyte cells such as mononuclear cells are preferred. Further, since the gene expression of leukemia cells is analyzed, those samples wherein more than 65% of cells are Philadelphia (Ph) chromosome-positive cells (judged by FISH analysis detecting bcr/abl fused gene).

Expression amount of each gene in the cells may be measured by measuring the amount of the mRNA of each gene in the cells, and measurement of the amount of mRNA may be carried out by a well-known methods. For example, as described in Example below, the expression amounts may be measured by preparing a DNA microarray on which equiamounts of cDNAs of the genes to be examined are immobilized; synthesizing, on the other hand, labeled cDNAs by synthesizing the cDNAs in the presence of a labeled nucleotide using the RNAs in the sample cells as templates; incubating the labeled cDNAs with the DNA microarray under hybridization conditions so as to hybridize the cDNAs with the cDNAs on the DNA microarray; and measuring the amount of the label in each spot on the DNA microarray after washing. The method for measuring the expression amount of each gene in the sample cells is not restricted to this method, and any of the other methods may be employed as long as the expression amount of each gene may be measured. For example, each RNA in the cells may be measured by realtime-detection RT-PCR method, Northern blot method or the like. In a preferred mode, since expression amounts of a relatively large number of genes are measured, a method in which the labeled cDNAs prepared from the sample cells are hybridized with the respective genes immobilized on a microarray, and the amounts of the respective labels are measured, which is described in Example below, is preferred. Here, "expression amount" is not necessarily an absolute amount, but may be a relative amount. The amount is not necessarily numerically presented, and the cases where, for example, a visual label such as a fluorescent label is used as the label, and the judgment is carried out based on visual observation, are within the definition of "measurement of expression amount".

The measured expression amount of each gene is then compared with the expression amounts of the non-responders having sensitivity to imatinib and non-responders who do not have sensitivity to imatinib. To carry out this, needless to say, it is necessary to preliminarily measure the expression amounts of each gene in known responders and non-responders. These amounts are compared with the expression amount of each gene in the sample cells. The comparison may be carried out by comparing the expression amount of each gene in the sample cells with the mean values of those of each gene in the known responders and non-responders, judging to which mean value the measured expression amount is close, and judging whether the result is statistically significant or not. However, to more accurately carry out the judgment, it is preferred to calculate prediction score (PS value) by a statistical method, and to judge the sensitivity to imatinib based on the prediction score. In the present specification, the term "comparison" not only involves to compare the values as they are, but also involves statistical processing on the measured expression amounts and the measured values of the known responders and non-responders. The method for calculating prediction score *per se* is known (T. R. Golub et al., Science 286, 531-7. (1999); T. J. MacDonald et al., Nat Genet 29, 143-52. (2001)). That is, each gene ($g_i$) votes for either responder or non-responder depending on whether the expression level ($x_i$) in the sample is closer to the mean expression level of responders or non-responders. The magnitude of the vote ($v_i$) reflects the deviation of the expression level in the sample from the average of the two classes:

$$V_1 = |x_i - (\mu_r + \mu_n) / 2|$$

In the above equation, $\mu_r$ and $\mu_n$ represent mean values of the expression amounts of responder group and non-responder group, respectively. The votes are summed to obtain total votes for the responder ($V_r$) and non-responder ($V_n$), and PS values are calculated as follows:

$$PS=((V_r-V_n)/(V_r+V_n)) \times 100,$$

As is apparent from this definition, the PS value is within the range between -100 and 100. In cases where the PS value is a positive number, the patient is judged to be a responder, and in cases where the PS value is a negative number, the patient is judged to be a non-responder. The larger the absolute value of the PS value, the higher the confidence of the judgment. As will be concretely described in Example below, expression amounts of the above-described genes (1) to (15), and of genes (1) to (30) were measured, and PS values were calculated. As a result,

whether the patient is responder or non-responder was able to be correctly judged without even one case exception based on whether the PS value was positive or negative, in all of the totally 22 cases including the 18 "learning cases" used for the selection of the genes, and 4 "test cases" used to confirm the correctness of the method of the present invention. Especially, when the above-described genes (1) to (15) were used, the absolute values of PS values were not less than 30 without even one exception. Since the larger the absolute value, the higher the probability, a judgment criterion ruling, for example, that when the absolute value is not less than 5, 20 or the like, the judgment is made, and when the absolute value is less than the cut-off value, the judgment is withheld, may be made.

By the above-described method, whether an examinee has sensitivity to imatinib or not, that is, whether the examinee is a responder or non-responder of the therapy by imatinib may be judged.

The drug to which sensitivity may be judged by the above-described method is not restricted to imatinib, and sensitivity to derivatives of imatinib, that is, the compounds represented by the above-described Formula [I] wherein the hydrogen atom(s) on one or a plurality of optional carbon atoms constituting the imatinib is(are) substituted by (a) substituent group(s), and which exhibit the similar pharmacological effect to that of imatinib, may also be judged. The number of substituent groups is not restricted, and preferably not more than 5, and examples of the substituent groups include $C_1$-$C_6$ lower alkyl groups, halogens, amino group, hydroxyl group, nitro group, carboxyl group and the like, especially $C_1$-$C_6$ lower alkyl groups, although the substituent groups are not restricted thereto. Imatinib or derivatives thereof may be in the form of a pharmaceutically acceptable acid addition salt. Examples of the pharmaceutically acceptable acid addition salts include mesylate, hydrochloride, sulfate, nitrate and the like, although the examples are not restricted thereto.

## Examples

The present invention will now be described by way of examples thereof. It should be noted that the present invention is not restricted to the following examples.

### Clinical-Pathological Findings of Samples

Peripheral blood samples with informed consent from 22 adult myeloid leukemia patients prior to treatment with imatinib were obtained. Each patient was then enrolled into a phase II study of imatinib for assessing anti-leukemia effect of imatinib. mRNA from eighteen samples in which more than 65% of cells had been positive for the Ph chromosome (judged by a FISH analysis detecting a bcr/abl fusion gene) prior to treatment were analyzed on the cDNA-microarray system (hereinbelow described) prepared by the present inventors. Sixteen patients with CML in chronic phase were treated with 400 mg/day ofimatinib (imatinib mesilate, Trademark "Glivec" produced by Novartis Pharmaceuticals, the dose is in terms of the dose of imatinib) and two patients in blast crisis were treated with 600 mg/day. The clinical response to imatinib was determined by cytogenetic criteria; that is, by the percentage of peripheral blood cells positive for Ph chromosome by the FISH analysis (B.J. Druker et al., N Engl J Med344, 1031-7(2001)). The 12 patients who showed major cytogenetic responses (less than 35% of cells remaining positive for the Ph chromosome) were classified as responders, whereas the six patients with more than 65% of cells still positive for the Ph chromosome after five months of imatinib treatment were considered non-responders. The remaining four were reserved to test the predictive scoring system later. Of the 22, two "learning" cases (the cases used for the construction of the prediction system described later) were in blast crisis phase and two "test" cases (the cases used for testing the correctness of the prediction system described later) were in accelerated phase. The cytogenetic responses of these four patients were analyzed within 12 weeks after starting of treatment because imatinib was clinically ineffective and discontinued within 12 weeks. As controls, a mixture of mononuclear cells from peripheral blood of 11 healthy volunteers was used.

### Preparation of cDNA Microarrays

The above-mentioned cDNA microarray system was prepared as follows: First, to obtain cDNAs to be spotted on a glass slide, RT-PCR was performed for each gene by a known method (H. Okabe et al., Cancer Res 61, 2129-37. (2001)). More particularly, cDNA microarrays on which 23,040 types of cDNAs selected from UniGene data base of National Cancer for Biotechnology Information were immobilized were prepared as follows: That is, polyadenylated RNAs (polyA$^+$ RNAs) (Clontech) obtained from 12 types of normal human organs (brain, heart, liver, skeletal muscle, small intestine, spleen, placenta, thyroid, fetal brain, fetal kidney, fetal lung and fetal liver) were used for the preparation of the cDNAs. RNAs were transcribed using an oligo(dT) primer and Superscript II reverse transcriptase (Life Technologies Inc). Using primers specific for each of the genes selected as described above, a region sizing 200 to 1100 bp containing no repeating sequence and no poly(A) in each gene was amplified. The PCR product was electrophoresed on agarose gel, and whether the product showed a single band of the expected size was checked. If it showed the single band, it was used for spotting. These PCR products were purified, and spotted on Type-7 glass slides (Amersham

Biosciences) using a microarray spotter (Microarray spotter Generation III (Amersham Biosciences)). Five different groups of slides (4608 cDNAs were immobilized on each slide of each group, and so totally 23,040 cDNAs were immobilized on the slides of 5 groups). On each slide, 52 types of house keeping genes and two types of negative control genes were also spotted.

The primer sets used for amplifying the above-described 77 types of genes in the PCR were as shown in Tables 5 to 7. In Tables 5 to 7, the gene Nos. indicate the Nos. shown in Tables 1 to 4 described above. The thermal cycle conditions of the PCR were as follows: That is, after the first denaturation at 95°C for 5 minutes, a thermal cycle consisting of 95°C for 30 seconds, 60°C for 30 seconds and 72°C for 1 minute was repeated 40 times, followed by the final treatment at 72°C for 10 seconds.

Table 5

| Gene (No., Symbol, GenBank ID) | Sequence | |
|---|---|---|
| | Forward Primer | Reverse Primer |
| (1)HN1(AI086871) | SEQ ID NO:1 | SEQ ID NO:2 |
| (2)AKR1C3(D17793) | SEQ ID NO:3 | SEQ ID NO:4 |
| (3)QARS(X76013) | SEQ ID NO:5 | SEQ ID NO:6 |
| (4)KIAA1105(AA136180) | SEQ ID NO:7 | SEQ ID NO:8 |
| (5)KIAA0668(AA506972) | SEQ ID NO:9 | SEQ ID NO:10 |
| (6)BLCAP(AF053470) | SEQ ID NO:11 | SEQ ID NO:12 |
| (7)ADFP(X97324) | SEQ ID NO:13 | SEQ ID NO:14 |
| (8)FLJ10422(AA894857) | SEQ ID NO:15 | SEQ ID NO:16 |
| (9)HMGCL(L07033) | SEQ ID NO:17 | SEQ ID NO:18 |
| (10)EST(AI051454) | SEQ ID NO:19 | SEQ ID NO:20 |
| (11)KLF4(A1290876) | SEQ ID NO:21 | SEQ ID NO:22 |
| (12)H3F3A(M11354) | SEQ ID NO:23 | SEQ ID NO:24 |
| (13)ACTB(V00478) | SEQ ID NO:25 | SEQ ID NO:26 |
| (14)DKFZP566D193(AA401318) | SEQ ID NO:2 | SEQ ID NO:28 |
| (15)APEX(U79268) | SEQ ID NO:29 | SEQ ID NO:30 |
| (16)DRIL1(U88047) | SEQ ID NO:31 | SEQ ID NO:32 |
| (17)BIN1(AF001383) | SEQ ID NO:3 | SEQ ID NO:3 |
| (18)EST(AA495984) | SEQ ID NO:35 | SEQ ID NO:36 |
| (19)CLTH(N41902) | SEQ ID NO:37 | SEQ ID NO:38 |
| (20)M6PR(AA179832) | SEQ ID NO:39 | SEQ ID NO:40 |
| (21)KIAA0106(D14662) | SEQ ID NO:41 | SEQ ID NO:42 |
| (22)IGF2R(J03528) | SEQ ID NO:43 | SEQ ID NO:44 |
| (23)IDH1(AA330014) | SEQ ID NO:45 | SEQ ID NO:46 |
| (24)EST(AI333449) | SEQ ID NO:47 | SEQ ID NO:48 |
| (25)SDHB(AA365986) | SEQ ID NO:49 | SEQ ID NO:50 |
| (26)TNRC3(AI743134) | SEQ ID NO:51 | SEQ ID NO:52 |

Table 6

| Gene (No., Symbol, GenBank ID) | Sequence | |
|---|---|---|
| | Forward Primer | Reverse Primer |
| (27)MGP(AA156488) | SEQ ID NO:53 | SEQ ID NO:54 |
| (28)CBLB(U26710) | SEQ ID NO:55 | SEQ ID NO:56 |
| (29)EST(AA055355) | SEQ ID NO:57 | SEQ ID NO:58 |
| (30)FLT10803(T70782) | SEQ ID NO:59 | SEQ ID NO:60 |
| (31)IMPDH1(J05272) | SEQ ID NO:61 | SEQ ID NO:62 |
| (32)FLJ20489(AI091459) | SEQ ID NO:63 | SEQ ID NO:64 |
| (33)GTF2I(U77948) | SEQ ID NO:65 | SEQ ID NO:66 |
| (34)CGI-57(AF070638) | SEQ ID NO:67 | SEQ ID NO:68 |

Table 6 (continued)

| Gene (No., Symbol, GenBank ID) | Sequence | |
|---|---|---|
| | Forward Primer | Reverse Primer |
| (35)LOC51312(.AI128538) | SEQ ID NO:69 | SEQ ID NO:70 |
| (36)CHAC(AA228874) | SEQ ID NO:71 | SEQ ID NO:72 |
| (37)ATP1B1(X03747) | SEQ ID NO:73 | SEQ ID NO:74 |
| (38)CPTIA(AA632225) | SEQ ID NO:75 | SEQ ID NO:76 |
| (39)CTSG(M16117) | SEQ ID NO:77 | SEQ ID NO:78 |
| (40)AXUD1(AI091372) | SEQ ID NO:79 | SEQ ID NO:80 |
| (41)HLA-B(M28204) | SEQ ID NO:81 | SEQ ID NO:82 |
| (42)GOLGA4(U31906) | SEQ ID NO:83 | SEQ ID NO:84 |
| (43)EST(AA743462) | SEQ ID NO:85 | SEQ ID NO:86 |
| (44)SCYA13(U46767) | SEQ ID NO:87 | SEQ ID NO:88 |
| (45)B4GALT1(D29805) | SEQ ID NO:89 | SEQ ID NO:90 |
| (46)DKFZP564O0463(AA143048) | SEQ ID NO:91 | SEQ ID NO:92 |
| (47)HSJ1(X63368) | SEQ ID NO:93 | SEQ ID NO:94 |
| (48)MRPL3(X06323) | SEQ ID NO:95 | SEQ ID NO:96 |
| (49)C9orf10(D80005) | SEQ ID NO:97 | SEQ ID NO:98 |
| (50)DDX1(X70649) | SEQ ID NO:99 | SEQ ID NO:100 |
| (51)EST(AA421326) | SEQ ID NO:101 | SEQ ID NO:102 |
| (52)HLA-A(AF055066) | SEQ ID NO:103 | SEQ ID NO:104 |

Table 7

| Gene (No., Symbol, GenBank ID) | Sequence | |
|---|---|---|
| | Forward Primer | Reverse Primer |
| (53)STIM1(AA101834) | SEQ ID NO:105 | SEQ ID NO:106 |
| (54)AMPD2(M91029) | SEQ ID NO:107 | SEQ ID NO:108 |
| (55)STX5A(U26648) | SEQ ID NO:109 | SEQ ID NO:110 |
| (56)IFNB1(M25460) | SEQ ID NO:111 | SEQ ID NO:112 |
| (57)MAEA(AI291745) | SEQ ID NO:113 | SEQ ID NO:114 |
| (58)LBR(L25941) | SEQ ID NO:115 | SEQ ID NO:116 |
| (59)LIMK2(D45906) | SEQ ID NO:117 | SEQ ID NO:118 |
| (60)EST(AI365683) | SEQ ID NO:119 | SEQ ID NO:120 |
| (61)RPL26(AA778161) | SEQ ID NO:121 | SEQ ID NO:122 |
| (62)FLJ10209(AL137271) | SEQ ID NO:123 | SEQ ID NO:124 |
| (63)FAAH(AA132519) | SEQ ID NO:125 | SEQ ID NO:126 |
| (64)C210RF33(Y07572) | SEQ ID NO:127 | SEQ ID NO:128 |
| (65)EST(Z44513) | SEQ ID NO:129 | SEQ ID NO:130 |
| (66)PRKACA(X07767) | SEQ ID NO:131 | SEQ ID NO:132 |
| (67)EPB49(L19713) | SEQ ID NO:133 | SEQ ID NO:134 |
| (68)EST(U51712) | SEQ ID NO:135 | SEQ ID NO:136 |
| (69)CRSP9(AI334396) | SEQ ID NO:137 | SEQ ID NO:138 |
| (70)EST(AA600323) | SEQ ID NO:139 | SEQ ID NO:140 |
| (71)STK22B(L77564) | SEQ ID NO:141 | SEQ ID NO:142 |
| (72)TRB@(X01410) | SEQ ID NO:143 | SEQ ID NO:144 |
| (73)EEF1D(Z21507) | SEQ ID NO:145 | SEQ ID NO:146 |
| (74)RPGR(U57629) | SEQ ID NO:147 | SEQ ID NO:148 |
| (75)ARRB1(AA918725) | SEQ ID NO:149 | SEQ ID NO:150 |
| (76)NOP5/NOP58(AA602490) | SEQ ID NO:151 | SEQ ID NO:152 |
| (77)IGL@(M87790) | SEQ ID NO:153 | SEQ ID NO:154 |

2. Measurement of Expression Amount of Each Gene

Using leukocyte cells prepared from peripheral blood of 11 healthy donors as a common control, gene expression analysis was carried out on the leukemia cells prepared from the peripheral blood of each CML patient. The preparation of the samples here was carried out as follows: That is, mononuclcar cells were prepared using Ficoll (Amersham Biosciences) and total RNAs were extracted using Trizol (Life Technologies, Inc, NY) according to the manufacturer's instructions. After treatment with DNase I (Nippon Gene, Tokyo Japan), T7-based RNA amplification method was carried out. This RNA amplification was carried out by the method of Luo, L. (Nat Med., 5; 117-122,1999). More particularly, this was carried out as follows: That is, reverse transcription reaction was carried out with Superscript II using the RNAs extracted from the sample and T7-oligo$(T)_{21}$ primer having T7 promoter sequence, to prepare single-stranded cDNAs. Then using the thus prepared single-stranded cDNAs as templates, reaction was carried out by DNA polymerase I using again the T7-oligo$(T)_{21}$ primer having T7 promoter sequence, to synthesize double-stranded cDNAs. Finally, after purifying the double-stranded cDNAs, RNA synthesis was carried out by T7 RNA polymerase using the double-stranded cDNAs as templates. Two rounds of amplification using 2 µg of total RNA as starting material yielded 40-100 µg of amplified RNA (aRNA). For control samples, two rounds of T7-based RNA amplification were also performed to obtain sufficient amounts of aRNAs. The amounts of the aRNAs were measured by spectrophotometry and their qualities were checked by modified agarose gel electrophoresis. RNAs amplified by this method accurately reflect the proportions in the original RNA source, as had been confirmed earlier by reverse transcription-polymerase chain reaction (RT-PCR) experiments, in which data from microarrays were consistent with results from RT-PCR whether total RNA or aRNA was used as the template (K. One et al., Cancer Res 60, 5007-11. (2000)).

Labeling, hybridization, washing, scanning, and quantification of signals were performed by the method described in K. One et al., Cancer Res 60, 5007-11. (2000) except that all processes were carried out with an Automated Slide Processor (H. Okabe et al., Cancer Res 61, 2129-37. (2001)). More particularly, these were carried out as follows; 2.5 µg of aRNA obtained from mononuclear cells in peripheral blood of healthy volunteer or CML patient was reverse transcribed as described above in the presence of Cy5-dCTP and Cy3-dCTP. The obtained labeled probes were mixed with microarray hybridization solution version 2 (Amersham Biosciences) and formamide (Sigma) to a final concentration of 50%. Hybridization and washing were carried out using a commercially available Automated Slide Processor (Amersham Biosciences) in accordance with the manufacturer's instruction. Then each of the fluorescent labels on the microarray were measured using Array Scanner Generation III (Amersham Biosciences).

3. Statistical Processing

First, genes were selected using the following two criteria; (i) signal intensities higher than the cut-off level in at least 80% of the cases; (ii) $|Med_r - Med_n| \geqq 0.5$ (where Med indicates the median derived from log-transformed relative expression ratios in responders or non-responders). Each hybridization signal intensity was optically evaluated by using a commercially available software (Array Vision computer program (Amersham Biosciences)), and normalized to the mean signal of the house keeping genes. By averaging the spots, CY3:CY5 ratio of each sample was calculated. The above-described cut-off value of the expression level was automatically calculated according to background fluctuation. The fluctuation may be evaluated by the value obtained by subtracting the variance of the log-transformed Cy3:Cy5 ratio of the highly expressed genes (top 30%. When the background fluctuation is small, it can be ignored) from the variance of the logarithmic ratio of Cy3:Cy5. Genes whose fluctuation is less than the critical value (1.0), and whose expression level is higher than about $10^5$ were employed. This is because that other genes whose expression level is low are buried in the background fluctuation. To compensate the non-uniformity in the amounts of the spots on the slide of microarray (although controlled in a certain range), a control was provided, and the data were analyzed in terms of expression amount of the sample compared to the control. The ratio obtained by dividing the expression amount in the sample by the expression amount in the control is called "relative expression amount ratio".

Then a permutation test to select genes that were useful for separation of the responder group from the non-responder group was carried out, This was carried out as follows: The mean (µ) and standard deviation (σ) were calculated from the log-transformed relative expression ratios of each gene in responder (r) and non-responder (n) patients. A discrimination score (DS) for each gene was defined as follows:

$$DS = (\mu_r - \mu_n) / (\sigma_r + \sigma_n)$$

Permutation tests to estimate the ability of individual genes to distinguish between responders and non-responders was carried out; samples were randomly permutated between the two classes 10,000 times. Since the DS dataset of each gene showed a normal distribution, a p-value for the user-defined grouping was calculated by a known method (T. R. Golub et al., Science 286, 531-7, (1999)). That is, by carrying out permutation test, normal distribution constituted

by the DS value for each gene is formed, and mean and standard deviation are calculated. Using this mean and standard deviation, p-value was calculated according to the equation.

As a result, 77 genes were listed as candidates that showed a permutation p-value of less than 0.05. Expression levels were increased for 33 of those genes and decreased for the other 44 in the non-responder group, as compared to the responder group.

Using this information, it was attempted to establish a scoring system to predict the efficacy of imatinib treatment. In accordance with a known method (T. R. Golub et al., Science 286, 531-7. (1999); T. J. MacDonald et al., Nat Genet 29, 143-52. (2001)), prediction score (PS) was calculated. That is, each gene ($g_i$) votes for either responder or non-responder depending on whether the expression level ($x_i$) in the sample is closer to the mean expression level of responders or non-responders in reference samples. The magnitude of the vote ($v_i$) reflects the deviation of the expression level in the sample from the average of the two classes:

$$V_i = |x_l - (\mu_r + \mu_n) / 2|$$

The votes were summed to obtain total votes for the responder ($V_r$) and non-responder ($V_n$), and calculated PS values as follows:

$$PS = ((V_r - V_n) / (V_r + V_n)) \times 100,$$

reflecting the margin of victory in the direction of either responder or non-responder. PS values range from -100 to 100; a higher absolute value of PS reflects a stronger prediction.

The 77 candidate genes were rank-ordered on the basis of the magnitude of their permutation p-values (Tables 1 to 4) and calculated the prediction score by the leave-one-out test for cross-validation using the top 5, 10, 15, 20, 25, 30, 35, 40, 45, 50, 55, 60, 65, 70, 75, and 79 genes on the rank-ordered list. This was carried out as follows: That is, one sample was left out, then permutation p-value and the mean expression level are calculated for the remaining samples, and then prediction score was calculated to determine the group of the left out sample. This operation was carried out for the respective 18 samples.

Then, to determine the number of discriminating genes that provided the best separation of the two groups, a classification score (CS) was calculated for each gene set. This was carried out as follows: That is, the classification score (CS) was calculated by using the prediction score of responders ($PS_r$) and non-responders ($PS_n$) in each gene set, as follows:

$$CS = (\mu_{PSr} - \mu_{PSn}) / (\sigma_{PSr} + \sigma_{PSn})$$

A larger value of CS indicates better separation of the two groups by the predictive-scoring system.

The number of genes used for calculation influences the power for separation of the two groups. The best separation was obtained when the top 15 or 30 genes in the candidate list shown in Tables 1 to 4 were used for calculation of the scores (Fig. 1). The "Prediction Score" system using these two sets of genes clearly separated the two patient groups (Fig. 2). Hierarchical clustering using the same gene sets was also able to classify the groups with regard to imatinib sensitivity (Fig. 3). A hierarchical clustering method was applied using the 15 and 30 highest-ranking (by permutations tests) discriminating genes. The analysis was performed using web-available software ("cluster" and "treeview") written by M. Eisen (http://genome-www5/stanford.edu/MicroArray/SMD/restech.html). Before the clustering algorithm was applied, the fluorescence ratio for each spot was first log-transformed and then the data for each sample were median-centered to remove experimental biases.

4. Judgment of Test Cases

To validate this prediction system, four additional ("test") cases that were completely independent from the 18 "learning" cases used for establishing the system were investigated by carrying out the above-described analysis using the total RNA in the mononuclear cells in peripheral blood as the starting material. Gene-expression profiles in each of these four samples were examined and then a prediction score was calculated for each of them using the above-described top 15 ((1) to (15)) or top 30 ((1) to (30)) discriminating genes (see Tables 1 to 4). As shown in Fig. 4, responsiveness of each of these four patients to imatinib was predicted with 100% accuracy.

## Claims

1. A method of judging sensitivity to imatinib or a derivative thereof or a pharmaceutically acceptable salt thereof, comprising measuring expression amounts of a plurality of genes selected from the group consisting of the following genes (1) to (77) in sample cells separated from body; and comparing the measured amounts with those of responders and non-responders to imatinib or a derivative thereof or a pharmaceutically acceptable salt thereof:

   (1)HN1(AI086871), (2)AKR1C3(D17793), (3)QARS(X76013), (4)KIAA1105(AAI36180), (5)KIAA0668(AA506972), (6)BLCAP(AF033470), (7)ADFP(X97324), (8)FLJ10422(AA894857), (9)HMGCL(L07033), (10)EST(AI051454), (11)KLF4(AI290876), (12)H3F3A(M11354), (13)ACTB(V00478), (14)DKFZP566D193(AA401318), (15)APEX(U79268), (16)DRIL1(U88047), (17)BIN1(AF001383), (18)EST(AA495984), (19)CLTH(N41902), (20)M6PR(AA179832), (21)KIAA0106(D14662), (22)IGF2R(J03528), (23)IDH1(AA330014), (24)EST(AI333449), (25)SDHB(AA365986), (26)TNRC3(AI743134), (27)MGP(AA156488), (28)CBLB(U26710), (29)EST(AA055355), (30)FLJ10803(T70782), (31)IMPDH1(J05272), (32)FLJ20489(AI091459), (33)GTF2I(U77948), (34)CGI-57(AF070638), (35) LOC51312(.AI128538), (36)CHAC(AA228874), (37)ATP1B1(X03747), (38)CPTIA(AA632225), (39)CTSG(M16117), (40)AXUD1(AI091372), (41)HLA-B(M28204), (42)GOLGA4(U31906), (43)EST(AA743462), (44)SCYA13(U46767), (45)B4GALT1(D29805), (46)DKFZP564O0463(AA143048), (47)HSJ1(X63368), (48)MRPL3(X06323), (49)C9orf10(D80005), (50)DDX1(X70649), (51)EST(AA421326), (52)HLA-A(AF055066), (53)STIM1 (AA101834), (54)AMPD2(M91029), (55)STX5A(U26648), (56)IFNB1(M25460), (57)MAEA(A1291745), (58)LBR(L25941), (59)LTMK2(D45906), (60)EST(AI365683), (61)RPL26(AA778161), (62)FLJ10209(AL137271), (63)FAAH(AA132519), (64)C21ORF33(Y07572), (65)EST(Z44513), (66)PRKACA(X07767), (67)EPB49(L19713), (68)EST(U51712), (69)CRSP9(AI334396), (70)EST(AA600323), (71)STK22B(L77564), (72)TRB@(X01410), (73)EEF1D(Z21507), (74)RPGR(U57629), (75)ARRB1(AA918725), (76)NOP5/NOP58(AA602490), (77)IGL@(M87790) (wherein the characters in parentheses after the symbol of each gene denote GenBank Accession No.)

2. The method according to claim 1, which is a method for judging sensitivity to imatinib or a pharmaceutically acceptable salt thereof.

3. The method according to claim 1 or 2, wherein the expression amounts of 10 to 35 genes among said genes are measured.

4. The method according to claim 3, wherein 10 to 35 genes from No. (1), in an ascending order, of said genes (1) to (35) are measured.

5. The method according to claim 4, wherein the expression amounts of said genes (1) to (15) or said genes (1) to (30) are measured.

6. The method according to any one of claims 1 to 5, wherein the step of comparing said measured expression amounts with those of the responders and non-responders comprises statistically calculating prediction score.

7. The method according to any one of claims 1 to 6, which is a method for predicting therapeutic effect of chronic mycloid leukemia by imatinib or a derivative thereof or a pharmaceutically acceptable salt thereof.

8. The method according to claim 7, wherein said sample cells are leukocyte cells.

9. The method according to claim 8, wherein said leukocyte cells are mononuclear cells.

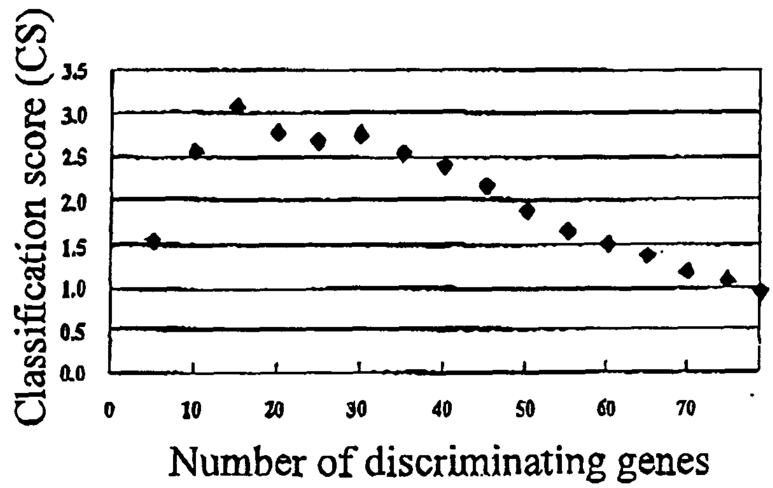
Classification score (CS)
3.5
3.0
2.5
2.0
1.5
1.0
0.5
0.0
0
10
20
30
40
50
60
70
Number of discriminating genes

Fig. 1

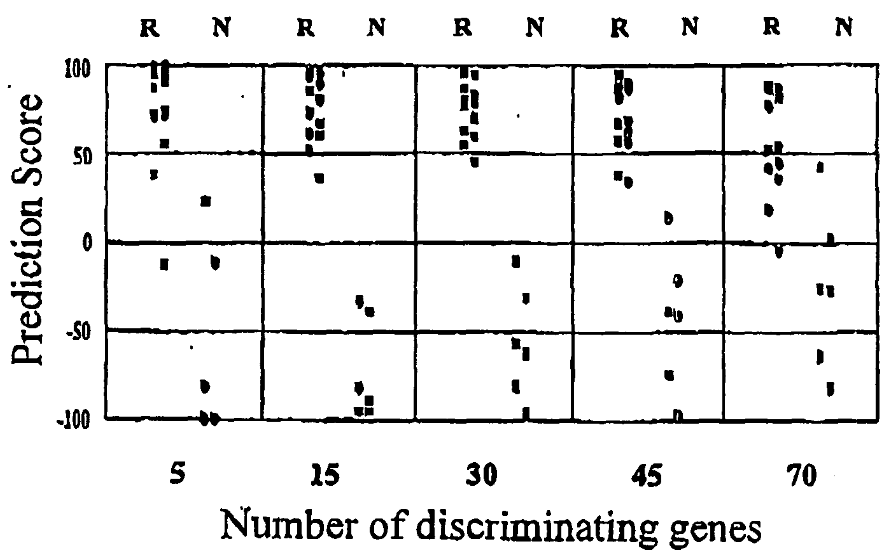
R N R N R N R N R N
Prediction Score
100
50
0
-50
-100
5
15
30
45
70
Number of discriminating genes

Fig. 2

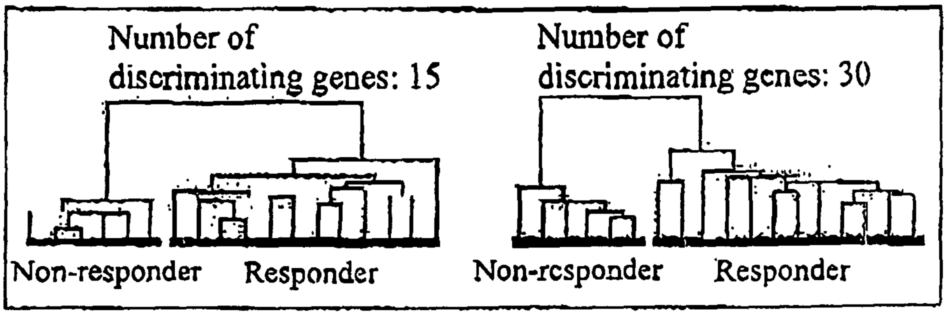
Number of discriminating genes: 15
Number of discriminating genes: 30
Non-responder
Responder
Non-responder
Responder

Fig. 3

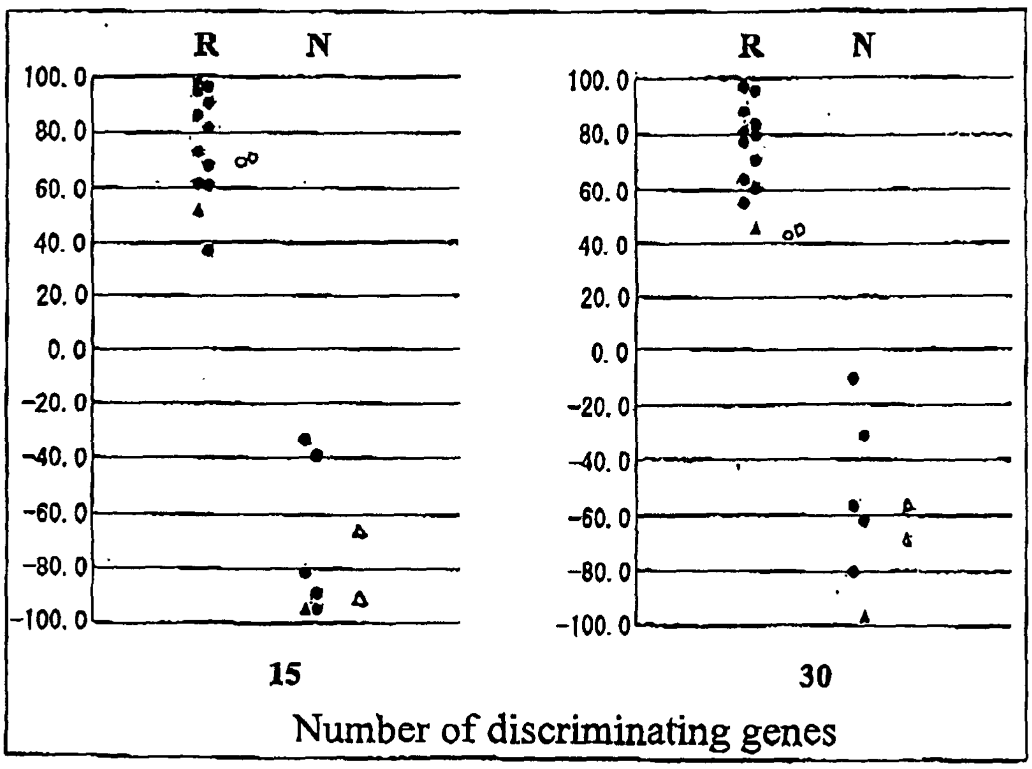
R
N
100.0
80.0
60.0
40.0
20.0
0.0
-20.0
-40.0
-60.0
-80.0
-100.0
15
30
Number of discriminating genes

Fig. 4

## INTERNATIONAL SEARCH REPORT

International application No.
PCT/JP03/06330

**A. CLASSIFICATION OF SUBJECT MATTER**
Int.Cl$^7$ C12N15/09, C12Q1/68

According to International Patent Classification (IPC) or to both national classification and IPC

**B. FIELDS SEARCHED**

Minimum documentation searched (classification system followed by classification symbols)
Int.Cl$^7$ C12N15/09, C12Q1/68

Documentation searched other than minimum documentation to the extent that such documents are included in the fields searched

Electronic data base consulted during the international search (name of data base and, where practicable, search terms used)
CA(STN), MEDLINE(STN), REGISTRY(STN), WPIDS(STN), BIOSIS/WPI(DIALOG)

**C. DOCUMENTS CONSIDERED TO BE RELEVANT**

| Category* | Citation of document, with indication, where appropriate, of the relevant passages | Relevant to claim No. |
|---|---|---|
| P,X | KANETA, Y et al., Prediction of sensitivity to STI571 among chronic myeloid leukemia patients by genome-wide cDNA micro-array analysis., Jpn.J. Cancer Res., 2002 August, Vol.93, No.8, pages 849 to 856 | 1-9 |
| A | HASHIMOTO, Y et al., A rescue factor abolishing neuronal cell death by a wide spectrum of familial Alzheimer's disease genes and Aβ, Proc.Natl.Acad. Sci.USA, 2001, Vol.98, No.11, pages 6336 to 6341 | 1-9 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 September, 2003 (03.09.03) | 16 September, 2003 (16.09.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06330

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

Concerning the claimed genes (1) to (77), there is no common chemical structure among the base sequences but the only one matter common to these genes resides in being usable in judging the sensibility to imatinib, its derivative or a pharmacologically acceptable salt thereof (hereinafter referred to as imatinib, etc.). However, use of the expression profiles of various genes in judging the sensibility to imatinib, etc. is reported in the following document. Thus, provision of genes usable in judging the sensibility to imatinib, etc. cannot be considered as a special technical feature in the meaning as described in PCT Rule 13.2. Such being the case, the genes (1) to (77) are considered not as relating (continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
The part HN1 (AI086871) in claim 1.

**Remark on Protest** ☐ The additional search fees were accompanied by the applicant's protest.
☐ No protest accompanied the payment of additional search fees.

Form PCT/ISA/210 (continuation of first sheet (1)) (July 1998)

INTERNATIONAL SEARCH REPORT

International application No.
PCT/JP03/06330

Continuation of Box No.II of continuation of first sheet(1)

to a group of inventions so linked as to form a single general inventive concept but as an invention group consisting of 77 inventions.

Document

1. Hofmann WK et al, Relation between resistance of Philadelphia-chromosome-positive acute lymphoblastic leukaemia to the tyrosine kinase inhibitor STI571 and gene-expression profiles: a gene-expression study., Lancet., 2002 Feb, Vol.359, No.9305, p.481-6

Form PCT/ISA/210 (extra sheet) (July 1998)